Europäisches Patentamt

European Patent Office

Office européen des brevets

(19)

(11) Publication number: **0 509 718 A1**

## EUROPEAN PATENT APPLICATION

(12)

(21) Application number: **92303208.0**

(22) Date of filing: **10.04.92**

(51) Int. Cl.⁵: **G01N 33/48**, G01N 33/53

(30) Priority: **12.04.91 US 684103**

(43) Date of publication of application:
**21.10.92 Bulletin 92/43**

(84) Designated Contracting States:
**AT BE CH DE DK ES FR GB IT LI LU NL SE**

(71) Applicant: **ORTHO DIAGNOSTIC SYSTEMS INC.**
**U.S. Route 202**
**Raritan New Jersey 08869(US)**

(72) Inventor: **Rao, Patricia E.**
**11 Pennlyle Road**
**Princeton Junction, New Jersey 08550(US)**

(74) Representative: **Mercer, Christopher Paul et al**
**Carpmaels & Ransford 43, Bloomsbury Square**
**London WC1A 2RA(GB)**

(54) Multiple colour staining reagent for blood cells and method of analysis.

(57) A multiple color staining reagent and method for blood cell analysis, especially through the use of flow cytometry, comprising two or more selected monoclonal antibodies coupled with a fluorescent label, particularly FITC and phycoerythrin, and an unlabeled, non-specific IgG antibody are provided. Specifically described is a multiple color staining reagent comprising a combination of two or more mouse monoclonal antibodies, each labeled with a different fluorescent dye, useful in double staining assays and the like for blood cell analysis.

EP 0 509 718 A1

## Background of the Invention

The present invention relates to a reagent and method of improving multiple color staining of blood for analysis using monoclonal antibodies, and more particularly, to substantially eliminate staining artifacts by adding non-specific mouse IgG to mouse monoclonal antibody preparations.

Preparations of two color reagents for blood cell analysis, such as flow cytometry, using mouse monoclonal antibodies coupled with fluorescein isothiocyanate (FITC) and Phycoerythrin (PE) have been commercially available for several years. However, currently marketed two color reagents, such as the Simultest™ series available from Becton Dickinson Company, are generally composed of multiple, mouse IgG2a antibodies. Mixtures of antibodies having IgG2a isotypes often produce data with significant staining artifacts when used as a staining reagent. Similar artifactual staining may be encountered when only one of the component antibodies has an IgG2a isotype. Such artifactual staining leads to errors in the interpretation of blood cell analysis, such as flow cytometry analysis.

Staining reagents comprising two or more labeled mouse IgG2a monoclonal antibodies or a combination of monoclonal antibodies having an IgG2a isotype with those having another isotype, or certain other combinations of isotypes may have specific clinical utility. For example, certain well-characterized antibodies with well known functional and clinical significance, such as Orthomune™ OKT3 (an anti-CD3 mouse monoclonal antibody) and OKT4a (an anti-CD4 mouse monoclonal antibody), both available from Ortho Diagnostic Systems Inc., would be useful in combination for a staining reagent, as this particular combination would allow clear identification of CD4(+) T cells and differentiate them from CD4(+) monocytes. One critical use of such combination staining reagents is in the evaluation of blood from patients infected with HIV-1 (AIDS). An accurate determination of the level of T cells becomes critical in diagnosis and drug therapy monitoring in this case. However, anti-CD4 antibodies, such as OKT4A and anti-CD3 antibodies, such as OKT3, have mouse IgG2a isotypes. Therefore, double-staining with these reagents may be complicated by artifactual staining. Accordingly, there is a need to substantially reduce the problem of artifactual double staining to render such combination monoclonal antibody reagents more accurate and easy to use.

In one approach to solving this problem that has often been encountered in the art, the present inventor successfully reduced artifactual double staining by purifying lymphocytes from other blood components including granulocytes, red blood cells, platelets, and plasma. However, this is not a practical solution to the problem since the intended use of multiple color reagents, such as the aforementioned two color reagents, is in the clinical lab in the analysis of whole blood.

A marked difference in the amount of artifactual double staining among whole blood samples collected with different anti-coagulants has also been observed. Blood samples collected in acid citrate dextrose (ACD) or sodium citrate anticoagulants manifested less artifactual double staining than those collected in anti-coagulants such as heparin or ethylenediaminetetra-acetic acid (EDTA). However, the selective use of a particular anticoagulant did not completely solve the problem of staining artifacts, and, in any event, would not prove to be clinically practicable, since it is current blood collection practice to use standard anticoagulants.

Accordingly, one object of the present invention is to provide a multiple color, combination monoclonal antibody staining reagent for blood cell analysis, especially flow cytometry analysis to avoid the aforementioned drawbacks. It is a further object of the invention to provide a reliable method to substantially reduce double staining artifacts. Another object of the invention is to provide a reagent which is useful for analysis of whole blood samples as well as whole blood that has been treated, such as "buffy coat" cells. Yet another object of the invention is to provide a reagent which is useful without substantial pre-processing of whole blood samples.

## Summary of the Invention

The present invention relates to a composition and method comprising multiple color staining reagents, useful in blood cell analysis such as fluorescent microscopy, flow cytometry, and the like. In a preferred embodiment, a multiple color reagent comprising at least two selected monoclonal antibodies coupled to different fluorescent labels, such as FITC and PE, and further comprising a non-specific IgG antibody is provided. A method for the preparation and use of multiple color reagents comprising adding non-specific IgG to selected monoclonal antibodies coupled with a combination of labels, such as FITC and PE is also provided. The invention is particularly useful when the selected monoclonal antibodies are mouse monoclonal antibodies, and are each of the isotype IgG2a.

## Brief Description of the Drawing

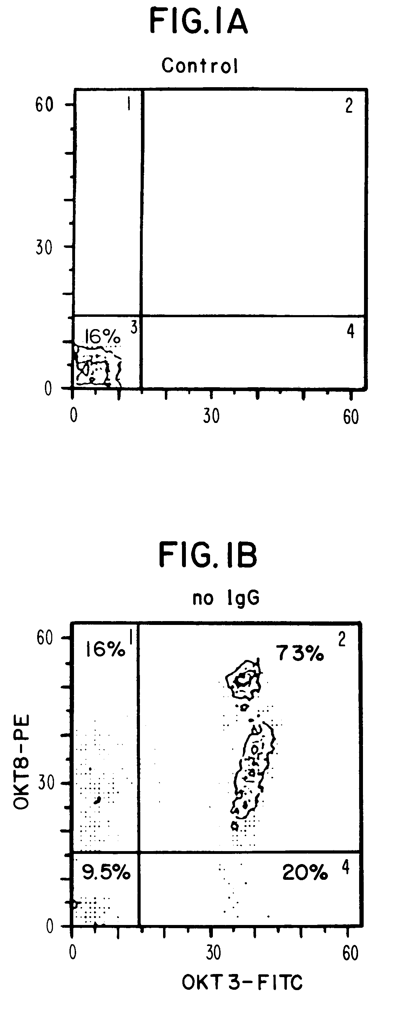

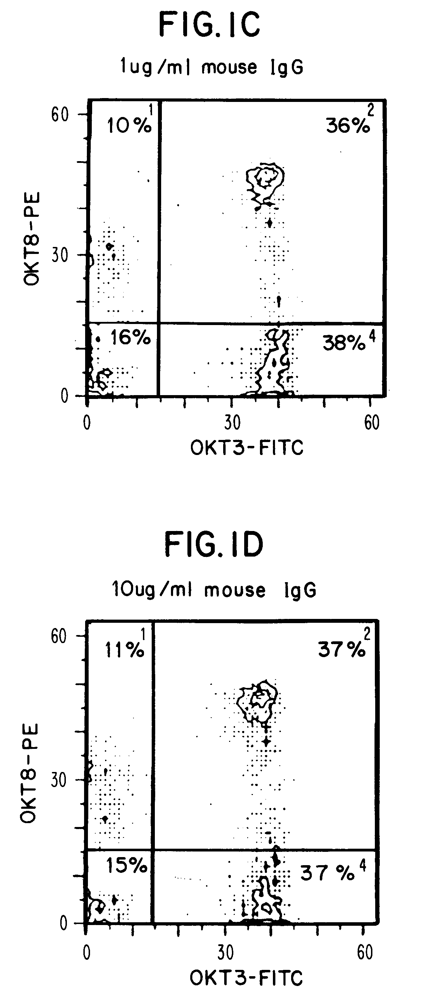

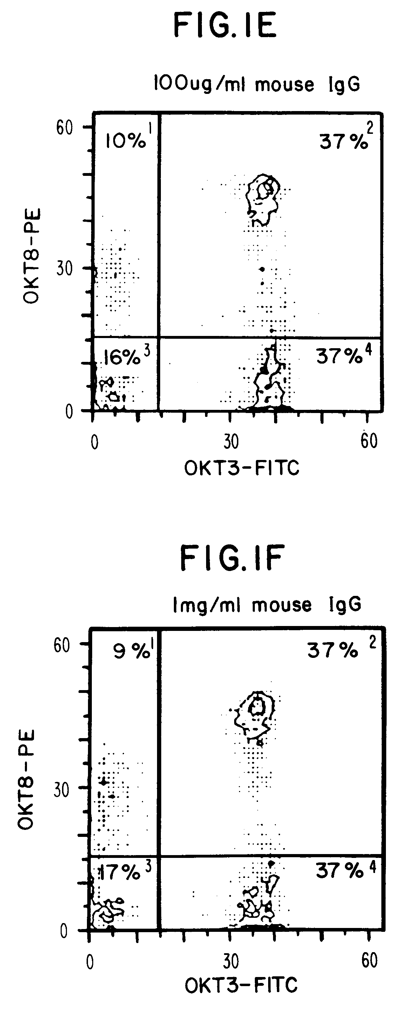

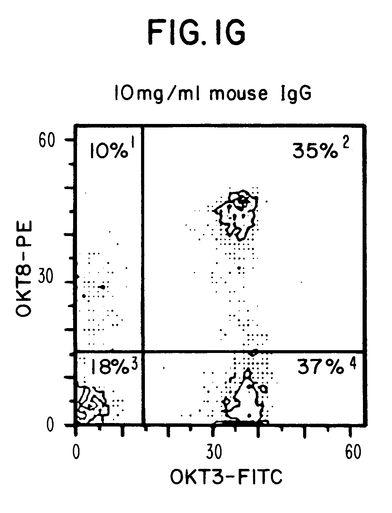

Figure 1 depicts eight two color plots obtained by flow cytometric analysis of whole blood stained with Orthomune™ OKT3 FITC and OKT8 PE (both available through Ortho Diagnostic Systems Inc.) with increasing amounts of mouse non-specific IgG. The first plot is a reference indicating the type of staining in each of the quadrants.

## Detailed Description of the Invention

The present invention comprises a multiple color staining reagent useful for the staining of blood cells for further analysis, such as fluorescent microscopy, flow cytometry, fluorescent methods applied to an adherent monolayer of cells, and the like. Monoclonal antibodies, having specificities of interest for reactivity with blood cells or parts thereof, are selected in accordance with a desired staining pattern of such blood cells, or parts of blood cells for analysis. The blood cell reactivity may be to blood cells from any mammalian species, with human blood cell reactivity generally the most useful for the reagent composition and methods described herein.

The monoclonal antibodies may be derived in whole or part from any suitable species such as hamster, rabbit, human, guinea pig, mouse and the like, or combinations thereof where suitable. The multiple color staining reagents as provided herein comprise a combination of two or more such monoclonal antibodies. The staining reagent combination preferably comprises two or more monoclonal antibodies derived from the same species, and most preferably two or more monoclonal antibodies derived from mouse.

As used herein, the term "monoclonal antibody" includes hybridoma derived antibodies, and all constructs of such antibodies, such as chimerics, hybrids, constructs comprising complementarity determining region grafting (CDR-grafted), fragments, repertoire cloned, and the like, whenever a combination of such antibodies would contain a combination of isotypes or parts thereof that foster artifactual staining when used as a staining reagent. A good description of such aforementioned various constructs may be found in U.S. patent # 4,816,567.

The antibodies may be obtained commercially from various sources such as Becton Dickinson and Company, New Jersey and Ortho Diagnostic Systems Inc., New Jersey. Alternatively, the antibodies may be prepared utilizing well-established techniques known to the art. Useful sources of such techniques may be found, inter alia, in Laboratory Techniques in Biochemistry and Molecular Biology - Monoclonal Antibody Technology, by Ailsa M. Campbell, Elsever, 1984, herein incorporated by reference.

Particularly useful for the purposes herein are combinations of two or more hybridoma-derived mouse monoclonal antibodies, especially those with human blood cell specificities.

Suitable amounts of monoclonal antibody in the reagent composition as provided herein are amounts sufficient to stain substantially all the nucleated cells present in volumes of blood typically used in clinical analysis such as about $20\mu l$ to about $300\mu l$ (per test tube). The ratio of the monoclonal antibody components used in these multiple color reagents is determined from the saturation titers of each of the individual antibody components. Saturation titers for the individual antibody components are determined using conventional staining techniques wherein a fixed number of cells is stained with a series of dilutions of an antibody component. The highest dilution which produces maximum fluorescent staining of substantially all cells in the population for which the antibody is specific is then determined as the saturation titer. In preferred embodiments, approximately twice the saturation titer of a particular antibody component is provided in the reagent composition.

The monoclonal antibodies contained in the composition are labeled with different select fluorescent labels generally available to the art. Differential labeling allows multiple staining assays to be conducted simultaneously, as cells bearing two different surface antigens can be enumerated in a single assay tube. This method also allows more specific identification of some cell types because cell populations bearing either one or both of the antigens can be visualized and enumerated. Suitable fluorescent labels as used herein include phycobiliproteins and chemical fluors such as XRITC, tetramethylrhodamine isothiocyanate (TRITC), FITC, and the like, and chlorophyll-based labels such as PerCP available from Becton Dickinson and Company. Particularly suitable for uses herein is a combination of labels that includes FITC and PE. Labeling of the monoclonal antibodies with such labels may be accomplished in accordance with well-known labeling techniques such as set forth in V. Oi, A.N. Glaser., L. Stryer (J. Cell Biol. 93:981 (1982)), and Weir, Herzenberg, Blackwell, Herzenberg, Handbook of Experimental Immunology, Vol. 1, Blackwell Scientific (1986), pp. 28.5-28.5, 31.4-31.7.

The reagent composition of the invention also comprises a quantity of unlabeled, non-specific IgG obtained from the same species from which at least one of the monoclonal antibody components are derived. This nonspecific IgG component may be polyclonal derived or monoclonal derived, and is present in quantities preferably ranging from about 0.001mg/ml to about 10mg/ml of reagent. Certain particularly preferred quantities range from about 0.01mg/ml to about 1mg/ml, as quantities in this preferred range satisfactorily correct most artifactual staining problems. The nonspecific IgG may be obtained commercially from a variety of vendors such as Calbiochem, in California. Additionally, one skilled in the art will understand that the nonspecific IgG may be prepared in accordance with well-accepted techniques such as those described in the Handbook of Experimental Immunology, supra.

In the reagent composition of the invention the monoclonal antibody and IgG components are suspended in any suitable buffer, such as phosphate buffer, TRIS buffer, phosphate buffered saline, and the like. One skilled in the art will understand that a suitable buffer is chosen in accordance with its ability to maintain the antibody combination in a stable and reactive form, fostering a suitable shelf life for the reagent. Components of the buffer must also be compatible with live cells. Additional components of the staining solution may include sodium azide, thimerisol and other suitable preservatives.

In a preferred method of blood cell analysis as provided herein, whole blood cell samples are collected using generally accepted clinical blood collection techniques. As is commonly the practice, anticoagulants such as EDTA, heparin, sodium citrate, acid citrate dextrose, or the like are employed.

Appropriate quantities of blood are pipetted into individual test tubes, generally with at least one test tube per antibody combination to be tested. If preformulated reagents containing non-specific IgG are not employed, then suitable quantities of IgG in an appropriate buffer, as described above, are added to each test tube. Suitable quantities of fluorescently coupled antibodies are also added to the test tube in a sequential fashion or together, and allowed to react with blood cells present in the sample. If commercially available monoclonal antibody reagents are utilized in this step, the manufacturer's suggested quantities should be used. One skilled in the art will, here again, also understand that such suitable reactant quantities are those that provide sufficient antibody to bind substantially all antigenic sites on the cells in the test tube, at or just above saturation as described above. Optional reaction times are about 20 to about 35 minutes at about 4°C.

Preferably, antibodies that have not reacted are removed by the addition of a suitable washing media, centrifugation and discarding of the supernatant fluid.

One skilled in the art will also appreciate that red blood cells present in a sample are generally lysed using a suitable lysing buffer such as Ortho Lysing Reagent™. It is understood that red blood cells overwhelm the field under analysis and render analysis of nucleated cell populations inaccurate. Optionally, nucleated cells are removing from the lysing media by centrifugation and are resuspended in a suitable quantity of media for cell analysis, such as in a flow cytometer.

In the particularly preferred embodiments, the blood collected in the first instance is washed with suitable cell media, such as PBS, Hank's Balanced Salt Solution, RPMI1640, and the like, prior to the addition of any of the reagents. If this wash step is performed, good results without artifactual staining may be achieved, even when anticoagulants such as EDTA have been used in the blood collection process.

In particularly preferred embodiments, a blood cell staining reagent kit and assay method is provided which comprises

    i) mouse monoclonal antibodies with selected blood cell specificities, coupled to a fluorescent label such as fluorescein isothiocyanate (FITC);
    ii) mouse monoclonal antibodies with certain other blood cell specificities coupled to a second fluorescent label, such as phycoerythrin (PE); and
    iii) a quantity of about 1mg/ml to about 0.01mg/ml of mouse nonspecific IgG.

The particularly preferred assay method comprises the use of the above composition for fluorescent analysis of cell surface antigens.

The following examples depict more specific aspects of the present invention, but should not be considered limitative thereof.

Example I

We attempted to determine the cause of artifactual double staining by evaluating (1) different anticoagulants, (2) the effect of lysing buffer, and (3) adding non-specific mouse IgG to the staining mixture. Purifying the lymphocytes on ficoll using conventional techniques prevented artifactual double staining. However, this approach is not practical for clinical use of a staining reagent, as such a reagent would generally be used with whole blood samples, buffy coat samples and the like. It could be inferred from these observations that the artifactual staining problem resulted from a blood component other than the lymphocytes themselves. The double staining artifact was also observed when the blood was processed without lysing buffer. Therefore, it could be inferred that the staining artifact was not caused by the conditions of lysis or the resulting debris.

Collection of blood in different anticoagulants, however, had a marked effect on the problem. Although always apparent, the problem was less severe when the blood was collected in acid citrate dextrose or sodium citrate. While the present inventor does not wish to be bound by theory, the ability of these anticoagulants to preserve platelets in a relatively unactivated state appeared to correlate with the reduction in artifactual staining.

The non-specific double staining was blocked in the following example by the addition of unlabeled, non-specific mouse IgG to the staining mixture comprising multiple mouse IgG2a antibodies coupled to FITC and PE.

100 $\mu$l of whole blood collected in heparin, or sodium citrate was added to test tubes. 100 $\mu$l of phosphate buffer solution (PBS) containing 1$\mu$g/ml, 10$\mu$g/ml, 100$\mu$g/ml, 1mg/ml, or 10mg/ml of non-specific, unlabeled mouse IgG was then added to the whole blood samples. Each sample was thereafter simultaneously stained with 10 $\mu$l of FITC and PE coupled with antibodies of clinical interest for 30 minutes on ice. Red cells were lysed with lysing reagent, Ortho Lysing Reagent™, available from Ortho Diagnostic Systems Inc., before flowcytometric analysis.

Stained cells were evaluated on the FACScan™ flowcytometer available from Becton Dickinson and Company. The results, shown in Figure 1, indicated that non-specific double staining of OKT3-FITC and OKT8-PE was eliminated by the addition of 1 or 10 $\mu$g/ml of non-specific mouse IgG to the staining mixture when heparin or sodium citrate was the anticoagulant (Figure 1C and 1D compared with Figure 1B). Additionally, double staining was somewhat improved in blood samples collected in EDTA when at least about 10mg/ml of non-specific mouse IgG was added to the staining mixture. The study population contained 76% OKT3( + ) cells and 41% OKT8( + ) cells.

The addition of non-specific mouse IgG to the formulation of FITC-PE IgG2a monoclonal reagents provides an effective method of substantially eliminating artifactual double staining in whole blood samples.

## Example II

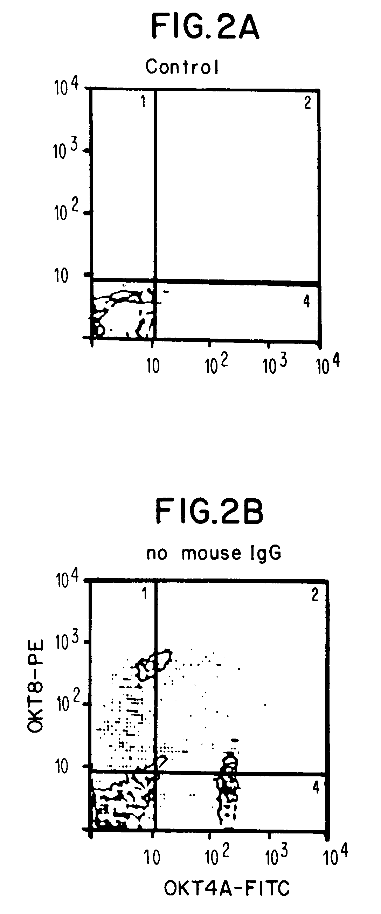

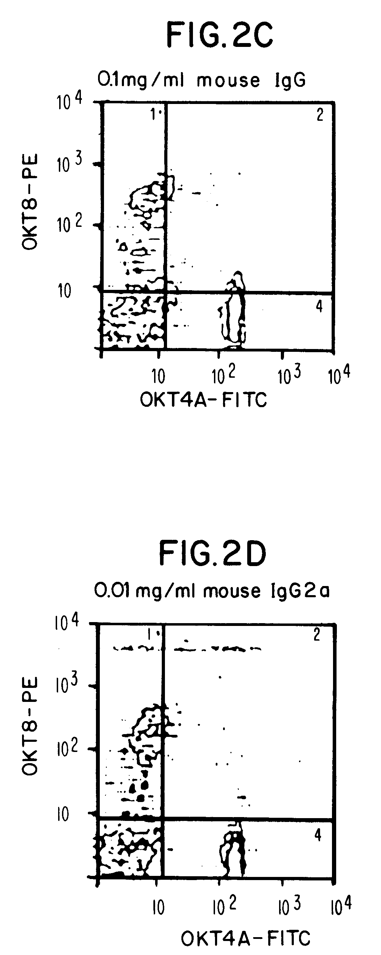

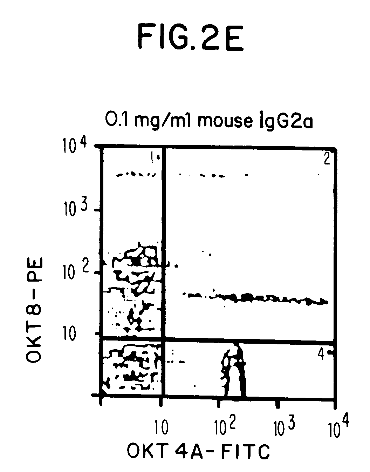

The effect of adding non-specific monoclonal mouse IgG2a to staining of heparinized whole blood with Orthomune™ FITC-OKT4A and PE-OKT8 is shown in Figure 2. Whole blood (100 $\mu$l) collected in heparin was combined with PBS containing 0.01mg/ml, or, 0.1mg/ml, of the non-specific mouse IgG2a. The non-specific mouse IgG2a was Ortho Control Antibody™ from Ortho Diagnostic Systems Inc. Reaction tubes then received 10 $\mu$l of FITC and PE coupled with the selected OKT antibodies of interest. Figures 2C and 2E demonstrate results using similar concentrations of non-specific mouse monoclonal IgG2a and polyclonal mouse IgG. Better correction of artifactual staining was obtained with the non-specific mouse monoclonal IgG2a at equivalent concentrations.

## Claims

1. A multiple color staining reagent for blood cells or fragments thereof, comprising two or more monoclonal antibodies each coupled with a different fluorescent dye, and an unlabeled non-specific IgG antibody of the same species as one of the two or more monoclonal antibodies.

2. The reagent of claim 1 wherein at least one of the two or more monoclonal antibodies is a mouse monoclonal antibody.

3. The reagent of claim 1 comprising at least about 1$\mu$g/ml of non-specific mouse IgG antibody.

4. The reagent of claim 3 wherein such non-specific mouse IgG is polyclonal derived.

5. The reagent of claim 3 wherein such non-specific mouse IgG is mouse monoclonal IgG2a.

6. The reagent of claim 3 wherein at least one of the antibody components is labeled with FITC and at least one other antibody component is labeled with PE.

7. A method for the preparation of a two color staining reagent for flow cytometry comprising adding non-specific mouse IgG antibody to a mixture of selected mouse monoclonal antibodies coupled with FITC and PE.

8. The method of claim 4 wherein at least two of the selected mouse monoclonal antibodies are of the IgG2a isotype.

# FIG.IA

## Control

# FIG.IB

## no IgG

# FIG. IC

1ug /ml mouse IgG

# FIG. ID

10ug /ml mouse IgG

# FIG.IE

### 100ug/ml mouse IgG

# FIG.IF

### Img/ml mouse IgG

# FIG. IG

10mg/ml mouse IgG

## FIG.2A

Control

## FIG.2B

no mouse IgG

OKT8-PE

OKT4A-FITC

# FIG.2C

0.1mg/ml mouse IgG

# FIG.2D

0.01 mg/ml mouse IgG2a

# FIG.2E

0.1 mg/ml mouse IgG2a

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8 505 640 (COULTER ELECTRONICS, INC.)<br>* abstract *<br>* page 7, line 28 - line 34; claims; examples 2,5,7 *<br>--- | 1,2,4 | G01N33/48<br>G01N33/53 |
| A | US-A-4 987 086 (BECTON,DICKINSON AND CO.)<br>* see the whole document, especially column 6, lines 58-62 *<br>--- | 1,7 | |
| A | EP-A-0 317 156 (BECTON,DICKINSON AND CO.)<br>* column 4, line 7 - line 36; claims *<br>--- | 1 | |
| A | INTERNATIONAL JOURNAL OF IMMUNOPHARMACOLOGY<br>vol. 3, no. 3, 1981, NEW YORK,USA<br>pages 249 - 254;<br>R.A. HOFFMAN ET AL.: 'Immunofluorescent analysis of blood cells by flow cytometry'<br>* abstract *<br>* page 251, left column, line 28 - right column, line 26 *<br><br>----- | 1 | TECHNICAL FIELDS SEARCHED (Int. Cl.5 )<br><br>G01N |

The present search report has been drawn up for all claims

| Place of search | Date of completion of the search | Examiner |
|---|---|---|
| THE HAGUE | 27 JULY 1992 | LUZZATTO E.R. |

EPO FORM 1503 03.82 (P0401)